Europäisches Patentamt

European Patent Office

Office européen des brevets

(11) Veröffentlichungsnummer: **0 360 119**
**A1**

## (12) EUROPÄISCHE PATENTANMELDUNG

(21) Anmeldenummer: 89116774.4

(22) Anmeldetag: 11.09.89

(51) Int. Cl.5: **C07C 303/06 , C07C 309/08 , C07C 309/10**

(30) Priorität: 19.09.88 DE 3831822

(43) Veröffentlichungstag der Anmeldung:
28.03.90 Patentblatt 90/13

(84) Benannte Vertragsstaaten:
**ES**

(71) Anmelder: **Henkel Kommanditgesellschaft auf Aktien**
**Henkelstrasse 67**
**D-4000 Düsseldorf 13(DE)**

(72) Erfinder: **Fabry, Bernd, Dr.**
**Danziger Strasse 31**
**D-4052 Korschenbroich 1(DE)**
Erfinder: **Westfechtel, Alfred, Dr.**
**Kerschensteiner Weg 9**
**D-4010 Hilden(DE)**

(54) **Verfahren zur Herstellung von Sulfonaten gegebenenfalls ethoxylierter und/oder propoxylierter Fettalkohole.**

(57) Ein Verfahren zur Herstellung von sulfonaten gegebenenfalls ethoxylierter und/oder propoxylierte Fettalkohole, bei dem Ester der Kohlensäure mit gegebenenfalls ethoxylierten und/oder propoxylierten Fettalkoholen mit 16 bis 22 Kohlenstoffatomen und mindestens einer olefinischen Doppelbindung sulfoniert und bei pH-Werten über 13 mit wässrigen Basen neutralisiert und hydrolysiert werden, ergeben oberflächenaktive Produkte mit niedrigem Salzgehalt.

EP 0 360 119 A1

EP 0 360 119 A1

## Verfahren zur Herstellung von Sulfonaten gegebenenfalls ethoxylierter und/oder propoxylierter Fettalkohole.

Die Erfindung betrifft Verfahren zur Herstellung von Sulfonaten gegebenenfalls ethoxylierter und/oder propoxylierter Fettalkohole.

Aus der DE-A 33 31 513 ist bekannt, daß hydrolysestabile Ethersulfonate durch Sulfonierung von Niedrigalkylethern ungesättigter Fettalkohole und Fettalkoholpolyalkylenglykolether durch Sulfonierung zugänglich sind. Die analoge selektive Herstellung sulfonierter ungesättigter Fettalkohole und Fettalkoholpolyalkylenglykolether ist infolge der in diesen vorhandenen freien OH-Gruppe nicht möglich.

In der nicht vorveröffentlichten deutschen Anmeldung P 37 25 030 vom 29. Juli 1987 ist ein Verfahren zur Herstellung von Sulfonaten gegebenenfalls ethoxylierter und/oder propoxylierter Fettalkohole beschrieben, bei dem die OH-Funktion der Fettalkohole, die innenständige olefinische Doppelbindungen aufweisen, zunächst durch Umsetzung mit einer $C_1$-$C_4$-Carbonsäure, insbesondere Essigsäure, geschützt wird. Nach der Sulfonierung wird das saure Umsetzungsprodukt alkalisch hydrolysiert, wobei eine quantitative Abspaltung der Esterschutzgruppe unter Rückbildung der freien terminalen OH-Funktion der Fettalkohole erfolgt. Bei diesem Verfahren bilden sich jedoch im Verlauf der Hydrolyse molare Mengen von Salzen der als Schutzgruppe eingesetzten $C_1$-$C_4$-Carbonsäuren, die das Produkt hinsichtlich Elektrolytgehalt und Umweltverträglichkeit (erhöhter chemischer Sauerstoffbedarf, z.B. durch Acetat) belasten.

Die nicht vorveröffentlichte deutsche Anmeldung P 38 24 720 vom 20. Juli 1988 beschreibt sulfonierte Ester der Kohlensäure mit gegebenenfalls ethoxylierten und/oder propoxylierten Fettalkoholen, wobei die Fettalkohole 16 bis 22 Kohlenstoffatome und mindestens eine innenständige olefinische Doppelbindung aufweisen. Diese Sulfonate haben sich als überraschend hydrolysestabil im pH-Bereich zwischen 6 und 13 erwiesen.

Die Erfindung beruht auf der Erkenntnis, daß sich die vorgenannten Sulfonate von Estern der Kohlensäure mit gegebenenfalls ethoxylierten und/oder propoxylierten Fettalkoholen in die freien, sulfonierten, gegebenenfalls ethoxylierten und/oder propoxylierten Fettalkohole überführen lassen. Dabei fällt bei der Hydrolyse von einem Mol Sulfonierungsprodukt nur ein halbes Mol der Esterschutzgruppe als Kohlendioxid an, das größtenteils als Gas entweicht und zu einem kleinen Teil als umweltunbedenkliches Carbonat gebunden wird. Es können somit Sulfonate von gegebenenfalls ethoxylierten und/oder propoxylierten Fettalkoholen mit niedrigem Elektrolytgehalt erhalten werden.

Demgemäß ist das Verfahren der Erfindung dadurch gekennzeichnet, daß man Ester der Kohlensäure mit gegebenenfalls ethoxylierten und/oder propoxylierten Fettalkoholen der allgemeinen Formel I

$$[RO-(C_mH_{2m}O)_n]_2 \ C=O \qquad (1)$$

in der
RO der Rest eines Fettalkohols mit 16 bis 22 Kohlenstoffatomen und mindestens einer olefinischen Doppelbindung,
m die Zahlen 2 und/oder 3 und
n eine Zahl im Bereich von 0 bis 20 bedeuten,
sulfoniert und bei pH-Werten über 13 mit wässrigen Basen neutralisiert und hydrolysiert.

Die erfindungsgemäß erhaltenen Verbindungen weisen interessante oberflächenaktive Eigenschaften auf.

Die Ausgangsprodukte für die Herstellung der Verbindungen der Erfindung, d.h. Fettsäureester der Kohlensäure, sind bekannt; sie lassen sich durch Umsetzung von Fettalkoholen mit Phosgen oder Chlorameisensäureester erhalten; vgl. J. Chem. Soc. 117, 708 (1920), J. Prakt. Chem. 22, 353 (1980). Eine noch einfachere Synthese beruht auf der Umesterung von Fettalkoholen mit Diethylcarbonat in Gegenwart alkalischer Katalysatoren; vgl. Chem. Ber. 114, 210 (1981), Houben/Weyl, Methoden der organischen Chemie, 4. Auflage, Bd. E4, S. 66 ff.. Analog lassen sich nach dem zuletztgenannten Verfahren Difettalkoholester oder gemischte Fettalkoholester der Kohlensäure herstellen.

In der allgemeinen Formel I bedeutet die Gruppe RO einen Rest eines Fettalkohols mit 16 bis 22 Kohlenstoffatomen und einer olefinischen Doppelbindung oder mehreren, wobei eine innenständige olefinische Doppelbindung oder zwei bevorzugt sind. Derartige Fettalkohole können synthetischer und insbesondere natürlicher Herkunft sein. Typische Vertreter geeigneter natürlicher Fettalkohole mit einer oder zwei olefinischen Doppelbindungen sind Oleyl-, Elaidyl-, Linoleyl-, Gadoleyl-, Arachidon-, Erucyl- und Brassidylalkohol. Wie in der Fettchemie üblich, können die Fettalkohole in Form ihrer an diesen reichen technischen Gemische mit anderen gesättigten oder ungesättigten Fettalkoholen eingesetzt werden, wie sie aus tierischen oder pflanzlichen Ölen und Fetten, z.B. Palmöl, Palmkernöl, Sojaöl, Rüböl, Olivenöl, Sonnenblumenöl, Korianderöl und dergleichen durch Hydrierung der in diesen enthaltenen Fettsäureester zugänglich

2

sind.

Entsprechend der für die allgemeine Formel I angegebenen Bedeutung für m = 2 und/oder 3 können in dem Verfahren der Erfindung auch Ester der Kohlensäure mit Anlagerungsprodukten von Ethylenoxid, Propylenoxid oder Ethylenoxid und Propylenoxid (in random- oder block-Verteilung der Propylenoxideinheiten) an ungesättigte Fettalkohole mit 16 bis 22 Kohlenstoffatomen bzw. technischen Gemischen derselben eingesetzt werden; die Gruppe RO kann mit 1 bis 20 Ethylenoxy- und/oder Propylenoxy-Gruppen versehen sein.

Gemäß einer vorteilhaften Ausführungsform der Erfindung verwendet man als Ausgangsprodukte Kohlensäureester der allgemeinen Formel I, in der

RO einen Oleyloxyrest,

m die Zahl 2 und

n eine Zahl im Bereich von 0 bis 10

bedeuten.

Als Sulfonierungsmittel können die für die Sulfonierung von Olefinen üblichen eingesetzt werden, z.B. Schwefelsäure, Chlorsulfonsäure, Oleum, Amidosulfonsäure oder Schwefeltrioxid, wobei - insbesondere gasförmiges -Schwefeltrioxid bevorzugt ist.

Die Sulfonierung von Kohlensäurefettalkoholestern der allgemeinen Formel I kann in der für Fettsäure-niedrigalkylester bekannten Weise erfolgen, z.B. mit gasförmigem Schwefeltrioxid in hierfür geeigneten Reaktoren, insbesondere vom Typ der Fallfilmreaktoren. Dabei wird das Schwefeltrioxid mit Luft oder Stickstoff verdünnt und vorzugsweise in Form eines Gasgemisches mit ca. 1 bis 8, insbesondere 3 bis 5 Vol.-% Schwefeltrioxid eingesetzt. Bevorzugt wird die Reaktion in Abwesenheit von Lösemitteln durchgeführt; es können jedoch auch sämtliche für die Sulfonierung ungesättigter Fettsäureester, Olefine, Aromaten und dergleichen üblichen Lösemittel eingesetzt werden.

Die Herstellung der Sulfonate gegebenenfalls ethoxylierter und/oder propoxylierter Fettalkohole der allgemeinen Formel I erfolgt bevorzugt durch Sulfonierung·mit 0,9 bis 1,8, insbesondere 1,0 bis 1,4 Mol gasförmigem Schwefeltrioxid pro Mol der in den Kohlensäureestern enthaltenen olefinischen Doppelbindungen bei Temperaturen von 15 bis 80, insbesondere 40 bis 60˚ C.

Das dabei erhaltene Sulfonierungsprodukt wird anschließend mit wässrigen Basen bei einem pH-Wert von mehr als 13 neutralisiert und hydrolysiert. Als Basen kommen hierbei Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak und organische Basen wie Ethanolamin, Diethanolamin oder Triethanolamin und primäre, sekundäre und tertiäre Alkylamine in Betracht. Die Basen kommen dabei vorzugsweise in Form mehr oder weniger konzentrierter Lösungen zum Einsatz. Ein bevorzugtes Hydrolysereagenz ist eine 25 bis 55 %-ige wässrige Natronlauge.

Gemäß einer weiteren vorteilhaften Ausführungsform der Erfindung erfolgt die Hydrolyse der Sulfonierungsprodukte bei Temperaturen von mindestens 70˚ C; die Hydrolysedauer kann zwischen 1 und 12 Stunden betragen. Dabei ist die Obergrenze der Hydrolysetemperatur durch den Siedepunkt des Wassers bestimmt. Man kann jedoch auch bei Temperaturen über 100˚ C unter Druck hydrolysieren, wobei sich dann die erforderlichen Hydrolysezeiten entsprechend verkürzen.

Wenn die eingesetzten Kohlensäureester der allgemeinen Formel I, wie bei der Herstellung aus technischen Fettsäureschnitten durchaus möglich ist, Anteile an Estern gesättigter Fettalkohole enthalten, können diese in den erfindungsgemäß hergestellten Sulfonierungsprodukten verbleiben oder, falls erwünscht, in an sich bekannter Weise, z.B. durch Phasentrennung, abgetrennt werden. Entsprechende Verfahren sind an sich für Sulfonate von Niedrigalkylestern ungesättigter Fettsäuren bekannt, vgl. die EP-A 0 130 753.

Bei der Sulfonierung der Kohlensäureester der allgemeinen Formel I können sich intermediär cyclische bzw. verbrückte Sulfonierungsprodukte bilden, die bei der erfindungsgemäß durchzuführenden Hydrolyse zerstört werden.

Die erfindungsgemäß hergestellten Sulfonate von gegebenenfalls ethoxyliertem und/oder propoxyliertem Fettalkohol können durch die im folgenden angegebene allgemeine Formel II beschrieben werden.

$RO\text{-}(C_mH_{2m}O)_n\text{-}O\text{-}H$     (II)

in der

RO eine Gruppe der Formeln IIIa bzw. IIIb

$CH_3\text{-}(CH_2)_x\text{-}CH(OH)\text{-}(CH_2)_y\text{-}CH(SO_3M)\text{-}(CH_2)_z\text{-}CH_2\text{-}O\text{-}$     (IIIa)

bzw.

$CH_3\text{-}(CH_2)_x\text{-}CH(SO_3M)\text{-}(CH_2)_y\text{-}CH(OH)\text{-}(CH_2)_z\text{-}CH_2\text{-}O\text{-}$     (IIIb)

in welcher

x und z Zahlen im Bereich von 0 bis 18,

3

y die Zahlen 0,1 oder 2 und

M ein Alkali-, Ammonium- oder gegebenenfalls mono-, di- oder trialkyl- bzw. hydroxyalkylsubstituiertes Ammoniumion ist,

wobei die Summe von x + y + z eine Zahl im Bereich von 14 bis 18 ergibt,

oder einen durch Abspaltung eines Moleküls Wasser aus der Gruppe der allgemeinen Formel IIIa bzw. IIIb gebildeter Rest ist,

m die Zahlen 2 und/oder 3 und

n eine Zahl im Bereich von 1 bis 20

bedeuten.

Die Erfindung wird im folgenden anhand bevorzugter Ausführungsbeispiele näher erläutert.

Aniontensid-Gehalt und die unsulfierten Anteile nach den DGF-Einheitsmethoden, Stuttgart 1950 - 1984, H-III-10 bzw. G-III-6b ermittelt. Die Bestimmung der Klett-Farbzahl erfolgte nach 30 min Bleichung mit 5 Gew.-%-iger Wasserstoffperoxidlösung. Die Messung erfolgte bei einer Konzentration von 5 Gew.-% Aniontensid, bei pH 7, unter Verwendung einer 1 cm-Rundküvette und eines Blaufilters (400 bis 465 nm).

Der Grad der Verseifung der Endprodukte wurde durch Bestimmung der Hydroxylzahl (OHZ) nach der DGF-Standardmethode sowie IR-spektroskopisch ($I_{CO}$) ermittelt, wobei für die letztere Methode die Intensität der CO-Schwingungsbande bei 1747 cm$^{-1}$ relativ zur CH-Adsorption bei 2920 cm$^{-1}$ herangezogen wurde.

Herstellung von Ausgangsverbindungen.

A. Bisoleyl-carbonat.

Es wurde ein technischer Oleylalkohol mit den folgenden Kenndaten eingesetzt:

C-Kettenverteilung:

$C_{14}$: 0 bis 2 %

$C_{16}$: 2 bis 10 %

$C_{18}$: 87 bis 95 %

$C_{20}$: 0 bis 3 %

Jodzahl: 90 bis 97

OH-Zahl: 205 bis 215.

In einem 1 l-Rundkolben mit Destillationsaufsatz wurden 268 g (1 mol) des vorgenannten Oleylalkohols und 59 g (0,5 mol) Diethylcarbonat vorgelegt und mit 1 g (entsprechend 0,5 mol-%) Natriummethylat in Form einer 30 %-igen methanolischen Lösung versetzt. Die Reaktionsmischung wurde eine Stunde auf 120°C erhitzt und anschließend 30 min bei 150°C gehalten. Freigesetztes Ethanol wurde abdestilliert. Anschließend wurde das Produkt 2 h bei 120°C im Wasserstrahlvakuum von Restmengen Alkohol befreit und mit Essigsäure neutralisiert.

Man erhielt ca. 320 g Produkt als hellgelbe, leicht bewegliche Flüssigkeit mit einer OH-Zahl von 9,5, einer Säurezahl von 0,1, einer Jodzahl von 48 und einem Zahlenmittel des Molekulargewichts von 548.

B. Bis(oleyl-5EO)carbonat.

478 g (1 mol) eines handelsüblichen Anlagerungsproduktes von 5 mol Ethylenoxid an 1 mol des oben genannten Oleylalkohols wurden mit 59 g (0,5 mol) Diethylcarbonat in der unter A. beschriebenen Weise umgesetzt. Man erhielt die Titelverbindung in einer Ausbeute von ca. 550 g als hellgelbe, trübe Flüssigkeit mit einer OH-Zahl von 8,5, einer Säurezahl von 0,1, einer Jodzahl von 31 und einem Zahlenmittel des Molekulargewichts von 905.

C. Bis(oleyl-10EO)carbonat.

707 g (1 mol) eines handelsüblichen Anlagerungsproduktes von 10 mol Ethylenoxid an ein Mol des oben beschriebenen technischen Oleylalkohols wurden mit 59 g (0,5 mol) Diethylcarbonat in der unter A. beschriebenen Weise umgesetzt. Man erhielt die Titelverbindung in einer Ausbeute von ca. 760 g als schwach gelb gefärbte, trübe Flüssigkeit mit einer OH-Zahl von 7,3, einer Säurezahl von 0,1, einer Jodzahl von 20 und einem Zahlenmittel des Molekulargewichts von 1402.

Beispiel 1.

Oleylalkohol-sulfonat.

a) Hydrolyse eines mit 20 %-igem Schwefeltrioxid-Überschuß hergestellten Produktes.

In einem 1l-Sulfierkolben mit Mantelkühlung und Gaseinleitungsrohr wurden 584 g (1 mol) Bisoleyl-carbonat vorgelegt und bei 40 bis 45°C mit 192 g (2,4 mol) gasförmigem Schwefeltrioxid umgesetzt.

Das Schwefeltrioxid wurde durch Erhitzen aus einer entsprechenden Menge 65 %-igen Oleums ausgetrieben, mit Stickstoff auf eine Konzentration von 5 Vol.-% verdünnt und innerhalb von 65 min in den Kohlensäurediester eingeleitet, wobei die Temperatur des Reaktionsgemisches durch Kühlung stets unter 60°C gehalten wurde.

Nach der Sulfonierung wurde das saure Reaktionsgemisch in eine Lösung von 200 g (5,0 mol) Natriumhydroxid in 500 ml Wasser eingerührt, 4 h bei 95°C und einem pH-Wert von 14 hydrolysiert und anschließend mit Mineralsäure auf einen pH von 7 eingestellt.

| Kenndaten des Produkts: | |
|---|---|
| Aniontensid(DGF-H-III-10) | 16,2 % = 0,0435 mval/g |
| Unsulfierte Anteile (DGF-G-III-6b) | 6,2 % |
| Sulfat (berechnet als $Na_2SO_4$) | 1,8 % |
| Carbonat (berechnet als $Na_2CO_3$) | 1,2 % |
| Wasser (nach Fischer) | 74,6 % |
| Klettfarbzahl | 102 |
| mittleres Molgewicht | 372 |
| OHZ | 151 |
| $I_{co}$ | 2 % |

b) Hydrolyse eines mit 40 %-igem Schwefeltrioxid-Überschuß hergestellten Produktes.

Der oben unter a) angegebene Ansatz wurde wiederholt, wobei die Umsetzung jedoch mit 224 g (2,8 mol) Schwefeltrioxid, entsprechend einem auf die Doppelbindungsäquivalente bezogenen 40 %-igem Schwefeltrioxid-Überschuß, durchgeführt. Das Schwefeltrioxid wurde unter den angegebenen Bedingungen innerhalb von 84 min eingebracht; das rohe Sulfierprodukt wurde mit 208 g (5,2 mol) Natriumhydroxid in 500 ml Wasser hydrolysiert und anschließend mit Mineralsäure auf einen pH-Wert von 6,5 bis 7,5 eingestellt.

| Kenndaten des Produkts: | |
|---|---|
| Aniontensid(DGF-H-III-10) | 24,0 % = 0,0645 mval/g |
| Unsulfierte Anteile (DGF-G-III-6b) | 1,8 % |
| Sulfat (berechnet als $Na_2SO_4$) | 2,1 % |
| Carbonat (berechnet als $Na_2CO_3$) | 1,0 % |
| Wasser (nach Fischer) | 71,1 % |
| Klettfarbzahl | 286 |
| mittleres Molgewicht | 372 |
| OHZ | 151 |
| $I_{co}$ | 2 % |

Beispiel 2.

Oleylalkohol-5EO-sulfonat.

In der in Beispiel 1 a) beschrieben Arbeitsweise wurden 496 g (0,5 mol) Bis(oleyl-5EO)carbonat mit 96 g (1,2 mol) Schwefeltrioxid sulfoniert und hydrolysiert.

| Kenndaten des Produkts: | |
|---|---|
| Aniontensid(DGF-H-III-10) | 16,4 % = 0,0277 mval/g |
| Unsulfierte Anteile (DGF-G-III-6b) | 6,4 % |
| Sulfat (berechnet als $Na_2SO_4$) | 2,0 % |
| Carbonat (berechnet als $Na_2CO_3$) | 1,0 % |
| Wasser (nach Fischer) | 74,2 % |
| Klettfarbzahl | 96 |
| mittleres Molgewicht | 592 |
| OHZ | 95 |
| $I_{co}$ | 2 % |

Beispiel 3.

Oleylalkohol-10EO-sulfonat.

In der in Beispiel 1 a) beschriebenen Arbeitsweise wurden 701 g (0,5 mol) Bis(oleyl-10EO)carbonat und 96 g (1,2 mol) Schwefeltrioxid umgesetzt und hydrolysiert.

| Kenndaten des Produkts: | |
|---|---|
| Aniontensid(DGF-H-III-10) | 18,8 % = 0,023 mval/g |
| Unsulfierte Anteile (DGF-G-III-6b) | 7,5 % |
| Sulfat (berechnet als $Na_2SO_4$) | 1,2 % |
| Carbonat (berechnet als $Na_2CO_3$) | 1,1 % |
| Wasser (nach Fischer) | 71,4 % |
| Klettfarbzahl | 41 |
| mittleres Molgewicht | 814 |
| OHZ | 70 |
| $I_{co}$ | 2 % |

## Ansprüche

1. Verfahren zur Herstellung von Sulfonaten gegebenenfalls ethoxylierter und/oder propoxylierter Fettalkohole, dadurch gekennzeichnet, daß man Ester der Kohlensäure mit gegebenenfalls ethoxylierten und/oder propoxylierten Fettalkoholen der allgemeinen Formel I

$[RO\text{-}(C_mH_{2m}O)_n]_2\ C = O$      (1)

in der

RO der Rest eines Fettalkohols mit 16 bis 22 Kohlenstoffatomen und mindestens einer olefinischen Doppelbindung,

m die Zahlen 2 und/oder 3 und

n eine Zahl im Bereich von 0 bis 20

bedeuten,
sulfoniert und bei pH-Werten über 13 mit wässrigen Basen neutralisiert und hydrolysiert.

II. Verfahren nach Anspruch 1, dadurch gekennzeichnet, daß man Kohlensäureester der allgemeinen Formel I verwendet, in der
RO einen Oleyloxyrest,
m die Zahl 2 und
n eine Zahl im Bereich von 0 bis 10
bedeuten.

III. Verfahren nach Anspruch 1 oder 2, dadurch gekennzeichnet, daß man die Kohlensäureester der allgemeinen Formel I mit Schwefeltrioxid, gegebenenfalls in inerten Lösemitteln, sulfoniert.

IV. Verfahren nach mindestens einem der Ansprüche 1 bis 3, dadurch gekennzeichnet, daß man die Kohlensäureester der allgemeinen Formel I mit 0,9 bis 1,8, insbesondere 1,0 bis 1,4 Mol gasförmigem Schwefeltrioxid pro Mol der in den Kohlensäureestern enthaltenen olefinischen Doppelbindungen bei Temperaturen von 15 bis 80, insbesondere 40 bis 60°C sulfoniert.

V. Verfahren nach mindestens einem der Ansprüche 1 bis 4, dadurch gekennzeichnet, daß man die Sulfonierungsprodukte der Kohlensäureester der allgemeinen Formel I bei Temperaturen von mindestens 70°C neutralisiert bzw. hydrolysiert.

VI. Verfahren nach mindestens einem der Ansprüche 1 bis 5, dadurch gekennzeichnet, daß man die Sulfonierungsprodukte der Kohlensäureester der allgemeinen Formel I mit 25 bis 55 %-iger Natronlauge neutralisiert bzw. hydrolysiert.

Europäisches
Patentamt

**EUROPÄISCHER RECHERCHENBERICHT**

Nummer der Anmeldung

EP 89 11 6774

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | DE-A-3 331 513 (HENKEL) --- | | C 07 C 303/06 C 07 C 309/08 C 07 C 309/10 |
| P,D A | DE-A-3 725 030 (HENKEL) ----- | | |

RECHERCHIERTE
SACHGEBIETE (Int. Cl.5)

C 07 C 309/00

Der vorliegende Recherchenbericht wurde für alle Patentansprüche erstellt

| Recherchenort | Abschlußdatum der Recherche | Prüfer |
|---|---|---|
| DEN HAAG | 16-11-1989 | ZAROKOSTAS K. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
...............................................................................
& : Mitglied der gleichen Patentfamilie, übereinstimmendes
   Dokument

EPO FORM 1503 03.82 (P0403)